(19) Europäisches Patentamt
European Patent Office
Office européen des brevets

(11) Publication number: 0 087 223
A1

(12) EUROPEAN PATENT APPLICATION

(21) Application number: 83300438.5

(22) Date of filing: 27.01.83

(51) Int. Cl.[3]: B 01 D 13/00
A 61 M 1/03, C 02 F 1/58

(30) Priority: 01.02.82 US 344747

(43) Date of publication of application:
31.08.83 Bulletin 83/35

(84) Designated Contracting States:
AT BE CH DE FR GB IT LI NL SE

(71) Applicant: UNION CARBIDE CORPORATION
Law Department - E134 Old Ridgebury Road
Danbury Connecticut 06817(US)

(72) Inventor: Sherman, John Delano
91 Valley View Road
Chappaqua New York 10514(US)

(72) Inventor: Ross, Ronald Jay
200 Oak Hill Drive
Upper Nyack New York 10960(US)

(74) Representative: McCall, John Douglas et al,
W.P. THOMPSON & CO. Coopers Building Church Street
Liverpool L1 3AB(GB)

# (54) Suppression of aluminum transport across dialysis membranes.

(57) There is disclosed a process whereby the addition of finely particulate silica to aqueous media containing dissolved aluminum species is found to inhibit the transport of the aluminum species across dialysis membranes. The procedure is particularly advantageous in preventing aluminum from entering the blood stream from the dialysate solution during hemodialysis.

EP 0 087 223 A1

11024/4

0087223



DESCRIPTION

"SUPPRESSION OF ALUMINUM TRANSPORT ACROSS DIALYSIS MEMBRANES"

This invention relates in general to the prevention of transport of aluminum or aluminum-containing molecular or ionic species across dialysis and similar membranes from aqueous solution containing same, and is especially useful in the prevention of aluminium transport across such membranes in the process of hemodialysis.

After years of relative unconcern among the medical professions about the potential toxicity of aluminum, a considerable amount of recently produced evidence leads toward the conclusion that the metal is in fact a neurotoxin in humans and other mammals. Elevated brain aluminum levels have been demonstrated in victims of Alzheimer's disease, in cases of progressive encephalopathy and in patients with dialysis dementia. Although the metabolism of aluminum has not yet been elucidated, it is now known that aluminum can enter the body during hemodialysis by transport of small particles of aluminum or aluminum-containing species across the dialysis membrane from the dialysate solution employed. Significant amounts of aluminum can be imparted to the dialysate from the water used to prepare it, from alumina supports for enzyme constituents and the like, and also from zeolitic aluminosilicates used as ion-exchange media.

Dialysis systems using crystalline aluminosilicate zeolites as ion-exchangers for ammonium cations are currently of special interest because of their high ammonium ion binding capacity and wide range of adaptability to meet the particular electrolyte requirements of individual patients. These zeolites, however, do represent a high potential for imparting

transportable aluminum species to the dialysate for a number of reasons. For example, zeolites are ordinarily prepared hydrothermally from reaction gels which are not 100% efficient in incorporating the available aluminum into the lattice of the crystallized product. It is inevitable that some of the unreacted amorphous alumina from the gel is occluded in the zeolite product and hence is capable of being readily leached out by contact with aqueous media. Also any dissolution of the zeolite crystal lattice itself can produce aluminum species soluble in aqueous media. Still further, attrition of zeolite agglomerates during the dialysis procedure can expose sources of soluble aluminum which are not reached during post-synthesis washing and purification of the zeolite product.

It would, therefore, be highly desirable to be able to reduce or entirely eliminate the number of aluminum species from aqueous media which are able to pass through conventional dialysis or ultrafiltration membranes, particularly those having pores of diameters in the range of about 10 to 15 Angstroms (allowing permeation of molecules with molecular weights up to the 1,000-2,000 range) which are suitable for hemodialysis.

It has now been found that such a result can be accomplished in a manner altogether compatible with conventional dialysis procedures by the process which comprises providing an aqueous medium in contact with a dialysis membrane which comprises :

(a) providing an aqueous medium in contact with a dialysis membrane and containing aluminum

species capable of passing through the pores of said membrane, and

(b) imparting thereto $SiO_2$ in the form of dispersed particles having particle sizes larger than the pores of said dialysis membrane and within the range of about 20 Angstrom to 40 micrometers, preferably 30 Angstrom to 40 micrometers, and more preferably within the range of 60 Angstrom to 30 micrometers, whereby the aluminum species become attached to the surfaces of the $SiO_2$ particles and form agglomerates too large to be transported through the said dialysis membrane.

As used herein, the term dialysis membrane is intented to mean any of the semipermeable films or other configurations which are characterized by having a large number of relatively uniform pores of molecular or near molecular dimensions which, under appropriate conditions of application, are able to function as a molecular sieve in the separation of molecules based on their size and shape. Such membranes are commonly used in the well-known ultrafiltration and electrodialysis procedures as well as the conventional passive dialysis in processes such as water desalination, the fractionation of proteins, membrane osmometry and the purification of municipal waste water. In general the membranes are prepared from natural or synthetic polymeric material by casting or extruding a solution of the polymer as a thin film. Recent improvements with respect to higher permeability coupled with higher permselectively, have been achieved using membranes formed from polyelectrolytes complexes, i.e. ionically crosslinked hydrogels formed by the reaction of two highly, but oppositely charged, polyelectrolytes. A concise

treatment of polyelectrolytes is contained in "Encyclopedia of Chemical Technology", Kirk-Othmer, 2nd Ed., Vol. 16, pgs 117-133, Interscience Pub. Although dialysis membranes as herein defined can have pore diameters ranging from about 10 Angstroms up to about 150 Angstroms, those of principal interest in the context of the present invention have pore diameters in the range about 10 Ansgtroms to 15 Angstroms for reasons which are apparent from the discussion of the $SiO_2$ particles below.

The term "aluminum species" as used herein is intended to mean monomeric or low molecular weight soluble or small colloidal forms of free aluminum ions, organoaluminum complexes, polymeric aluminum species and aluminosilicate species of the types which are formed in aqueous media containing dissolved aluminum salts, particularly those commonly found in aqueous media which have been in contact with elemental aluminum, aluminum oxides or aluminosilicates such as zeolites.

The source of the silica particles employed is not a critical factor, provided however, that their size is within the range of about 20 Ångstroms to about 40 micrometers. Particles within this size range, in addition to being too large to pass through the pores of dialysis membranes, are suspendable in aqueous media and are therefore able effectively to contact the aluminum species which form part of the solute of the dialysate. Preferred forms are aqueous colloidal silica sols such as those commercially available under the trade names

Ludox (E.I. DuPont du Nemours) and Nalcoag (Nalco Chemical Co.) and large particle size forms of precipitated silica, such as those commercially available under the trade name Syloid (W.R. Grace Co.). It should be noted however that silica sols are commonly stabilized toward gelling by one of several techniques which include the surface reaction of the individual $SiO_2$ particles with polyvalent metal ions, including aluminum ions, as described in detail in U.S. Patent No. 2,913,419 issued November 17, 1959 to G.B. Alexander and U.S. Patent No. 2,892,797 issued June 30, 1959 to G.B. Alexander et al. Since $SiO_2$ particles whose surfaces are not free to interact with additional aluminum species in an aqueous media will not be effective in the process of the present invention, care should be taken to avoid silica sols highly stabilized with polyvalent metal ions. Sols moderately stabilized in this manner are still effective in the present process, however, and are not to be excluded on that account.

The aluminum-containing aqueous medium suitably employed is also not critical with respect to its composition provided of course that it does not contain constituents which are destructive toward the dialysis membrane and/or the silica particles. Suitable media include the simple common tap water, which almost universally contains dissolved aluminum salts along with calcium, iron and magnesium salts and any of a variety of organic materials, up to the highly complex dialysate solutions used in hemodialysis. These latter solutions are exemplified by the commercial dialysate specified below in Example 1.

The medium can be a true solution or contain additional non-soluble constituents in the form of a slurry. In all cases liquid water should be present in sufficient amounts to represent the matrix component and permit the easy contact of the aluminum species with the silica particles. Neither the temperature nor the pressure conditions are critical provided the media remains in the liquid phase. The pH of the medium should generally be within 5 to 9, preferably 6 to 8.

The operation of the process of this invention is exemplified in the following examples :

Example 1

The effectiveness of the process of the present invention in removing aluminum imparted to an aqueous commercial dialysate solution by contact with zeolitic aluminosilicates was demonstrated by the following procedures :

(a) An aqueous slurry was prepared by adding approximately 100 grams of finely divided crystals of the sodium form of zeolite F having a chemical composition in terms of mole ratios of oxides (anhydrous basis)

$$(0.01K_2O + 0.94\ Na_2O): Al_2O_3\ 2\ \ SiO_2$$

to 0.5 liters of an aqueous dialysate solution containing the following constituents in the concentrations indicated below :

| | |
|---|---|
| Sodium chloride | 5.7 gms./liter |
| Sodium acetate | 3.0 gms./liter |
| Calcium chloride $.2H_2O$ | 0.26 gms./liter |
| Potassium chloride | 0.15 gms./liter |
| Magnesium chloride $\cdot 6H_2O$ | 0.15 gms./liter |
| Dextrose | 2.5 gms./liter |

The resultant slurry was mixed vigorously in a Waring Blendor for 15 minutes and a portion thereof was then removed. The remaining portion was mixed for an additional 15 minutes. The supernatant liquid from each portion was then separately filtered through a commercially available dialysis membrane having pore diameters of approximately 19 Angstroms. The filtered solutions were then separately admixed with silica particles by the addition of 2.37 grams of Syloid 63, a commercial silica having average particle sizes of about 9 micrometers and a surface area of about 675 $m^2/g$. (Al content = 467 micrograms/gram), and the resultant mixture blended for 15 minutes at moderate speed and again filtered through the dialysis membrane of the same type as previously employed. In each instance the filtrates resulting from passage through the dialysis membrane were analyzed for aluminum content. The results are set forth in Table A below :

(b) Using the same materials specified in part (a) supra, a slurry was prepared by combining all of the ingredients, i.e. the dialysis solution, the zeolite F and the silica, and blending the slurry for 15 minutes. At the end of this period a portion of the slurry was removed, and the remainder blended for an additional 15 minutes. The supernatant liquid from each sample was filtered through the same dialysis membrane as in part (a) and the filtrate analyzed for aluminum. The results are set forth in Table A below :

TABLE A

| Example | Filtrate Description | Aluminum Content mg./liter |
|---|---|---|
| 1-a-1 | Dialysate + zeolite F, blended 15 minutes | 0.40 |
| 1-a-2 | Dialysate + zeolite F, blended 30 minutes | 0.61 |
| 1-a-3 | Filtrate of (a-2) + silica, blended 15 minutes | 0.53 |
| 1-b-1 | Dialysate + zeolite F + silica blended 15 minutes | 0.70 |
| 1-b-2 | Dialysate + zeolite F + silica blended 30 minutes | 0.47 |

Example 2

The procedure of Example 1 was repeated using the same materials specified therein with the sole exception that the zeolite F constituent was substituted with the calcium form of zeolite W. This zeolite, in the anhydrous form, had the composition in terms of mole ratios of oxides as

$$(0.70\ CaO + 0.05\ K_2O + 0.25\ Na_2O) + Al_2O_3 + 3.6\ SiO_2$$

the results are set forth in Table B, below :

TABLE B

| Example | Filtrate Description | Aluminum Content mg./liter |
|---|---|---|
| 2-a-1 | Dialysate + zeolite W, blended 15 minutes | 0.15 |
| 2-a-2 | Dialysate + zeolite W, blended 30 minutes | 0.23 |
| 2-a-3 | Filtrate of (b-2) + Silica, blended 15 minutes | 0.07 |

| | | |
|---|---|---|
| 2-b-1 | Dialysate + zeolite W + Silica, blended 15 minutes | 0.07 |
| 2-b-2 | Dialysate + zeolite W + Silica, blended 30 minutes | 0.15 |

Example 3

(a) An aluminum-containing dialysate solution was prepared by adding aluminum chloride to the same dialysate solution as specified in Example 1. The resultant solution contained approximately 15 ppm Al. A portion of this solution was filtered through the same ultrafiltration membrane as employed in Example 1 and the filtrate analyzed for aluminum content. Another 500 ml portion of the aluminum-containing solution was admixed with 13.3 g. of a commercial silica. Nalcoag 1115, containing 15% $SiO_2$ with an average particle size of about 4 micrometers and a surface area of 750 m/g.) mixed for 30 minutes, blended, and filtered through the same dialysis membrane. The concentration of aluminum in the two filtrates is set forth in Table C below :

(b) The procedure of part (a) of this Example 3 was repeated using the same constituents except that the silica employed was 4.84 grams of a precipitated silica (Syloid 244 FP, having a particle size of approximately 4 micrometers and a surface area of 310 $m^2$/g.). The analytical results are shown in Table C below :

TABLE C

| Example | Filtrate Description | Aluminum Content mg./Liter |
|---|---|---|
| 3-a-1 | Dialysate + $AlCl_3$, blended 15 minutes | 0.55 |
| 3-a-2 | Filtrate of (3-a-1) +Silica blended 30 minutes | 0.11 |

| | | |
|---|---|---|
| 3-b-1 | Dialysate + $AlCl_3$ blended 15 minutes | 0.58 |
| 3-b-2 | Filtrate of (3-b-1) + Silica blended 30 minutes | 0.05 |

In each of the above examples the quantity of silica particles had a total surface area of approximately 1500 square meters in contact with a solution volume of about 500 ml., i.e., a proportion of about 300 $m^2$ per 100ml. solution. The presence of the silica particles reduced the amounts of aluminum species which could pass through the ultrafiltration membrane significantly, but did not reduce these amounts to zero. The addition of larger quantities of silica particles would, of course, further reduce the amounts of aluminum species which can pass through the membrane.

Similarly, the presence of silica particles in the slurry of dialysate solution plus zeolite reduced the quantities of aluminum species which could pass through the ultrafiltration membrane in comparison with the quantities passed when no silica particles were added. The addition of larger quantities of silica particles would further reduce the aluminum species passing through the membrane.

..................................................

CLAIMS

1. Process for inhibiting the transport of aluminum species from an aqueous medium through a dialysis membrane characterised by(a) providing an aqueous medium in contact with a dialysis membrane and containing aluminum species capable of passing through the pores of said membrane, and (b) imparting thereto $SiO_2$ in the form of dispersed particles having particle sizes larger than the pores of said dialysis membrane and within the range of 20 Angstroms and 40 micrometers.

2. Process according to claim 1, characterised in that the particle size of the particulate $SiO_2$ is within the range of 30 Angstroms to 40 micrometers.

3. Process according to claim 1 or 2, characterised in that the particle size of the particulate $SiO_2$ is within the range of 60 Angstroms to 30 micrometers.

4. Process according to any one of the preceding claims, characterised in that the aqueous medium is a dialysate suitable for use in hemodialysis.

.......................................................

0087223

European Patent Office

# EUROPEAN SEARCH REPORT

Application number

EP 83 30 0438

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | WASSERWIRTSCHAFT-WASSERTECHNIK, vol. 8, August 1958, Berlin-Ost W. CHRIST et al. "Über die Anwendung aktivierter Kieselsäure als Fällhilfsmittel in der Wasserreinigung", pages 361-364 * Complete document * --- | 1 | B 01 D 13/00<br>A 61 M 1/03 //<br>C 02 F 1/58 |
| A | Soviet Invention Illustrated Week D37, 21 October 1981, Section D15 & SU - A - 789 416 --- | 1 | |
| P,A | Patent Abstracts of Japan vol. 6, no. 73, 8 May 1982 & JP-A-57-10385 --- | 1 | |
| A | DE-A-1 642 837 (INSTITUT NATIONAL DU VERRE) * Claims 1, 7 * --- | 1 | TECHNICAL FIELDS SEARCHED (Int. Cl. ³) |
| A | EP-A-0 003 327 (PASSAVANT-WERKE MICHELBACHER HÜTTE) * Claim 1 * --- | 1 | A 61 M 1/03<br>B 01 D 13/00<br>B 01 D 21/01<br>B 01 J 20/10<br>C 02 F 1/44<br>C 02 F 1/58 |
| A | EP-A-0 005 193 (SARTORIUS) * Claim 1 * --- -/- | | |

The present search report has been drawn up for all claims

| Place of search | Date of completion of the search | Examiner |
|---|---|---|
| BERLIN | 29-04-1983 | KUEHN P |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

0087223

Application number

EP 83 30 0438

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,A | US-A-2 892 797 (G.B. ALEXANDER et al.)<br>* Claims 1, 2 *<br>--- | | |
| D,A | US-A-2 913 419 (G.B. ALEXANDER)<br>* Claim 1 *<br>----- | | |
| | | | TECHNICAL FIELDS SEARCHED (Int. Cl. 3) |

The present search report has been drawn up for all claims

| Place of search | Date of completion of the search | Examiner |
|---|---|---|
| BERLIN | 29-04-1983 | KUEHN P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82